# DEMANDE DE BREVET EUROPEEN

(11) **EP 0 532 418 A1**
(43) Date de publication de la demande: **17.03.1993**
(21) Numéro de dépôt: 92402481.3
(22) Date de dépôt: 11.09.1992
(51) Int. Cl.: A61L 2/06

(54) **Groupe mobile de stérilisation pour la matière microgranuleuse d'un lit fluidisé**

(30) Priorité: 11.09.1991 FR 9111355
(71) Demandeur: Wackermann, Guy, F-67100 Strasbourg (FR); Wackermann, Pierre, F-67200 Strasbourg (FR)
(72) Inventeur: Wackermann, Guy, F-67100 Strasbourg (FR); Wackermann, Pierre, F-67200 Strasbourg (FR)

(57) **Abrégé**

Groupe mobile de stérilisation pour la matière microgranuleuse d'un lit fluidisé sous la forme d'un caisson mobile à compartiment bas (5) et haut (6), le compartiment bas renfermant un groupe frigorifique (16) et une pompe de transfert (11) en liaison par conduit souple à couplage rapide avec un ensemble mobile d'aspiration sur le lit, le compartiment haut (6) délimitant une cuve de stérilisation (23) à fond (24) perméable à l'air dans laquelle est apportée la matière microgranuleuse par la pompe de transfert (11) et à l'arrière un volume aéraulique (27) présentant des moyens de conditionnement et de soufflage de l'air à travers la masse de matière à stériliser.

Cette invention intéresse les constructeurs de matériel médical.

## Description

La présente invention se rapporte à un groupe mobile de stérilisation en continu d'un produit pulvérulent ou microgranuleux.

Cette invention s'applique plus particulièrement à la stérilisation des microsphères utilisées comme matière en suspension dans les lits dits fluidisés utilisés en milieu hospitalier, notamment dans les centres de soins des grands brûlés.

Pour procurer l'effet recherché, ces lits contiennent cette matière microgranuleuse, en mouvement permanent généré par un fluide sous pression.

Le patient est isolé de ce matelas de matières en mouvement par un drap-filtre ou une alèse perméable à l'air.

Or, les caractéristiques de la matière composant cette protection se modifiant légèrement dans le temps, les propriétés de propreté bactériologique ne peuvent être garanties au-delà d'une certaine durée.

Ainsi, des exsudats liquides ou de matières organiques arrivent à contaminer la matière en suspension en traversant le drap-filtre puis la couche des microsphères en mouvement.

Les souillures ambiantes et celles provenant des exsudats du sujet constituent autant de sources d'infection.

En raison de la difficulté de transfert de cette importante masse de microsphères, on procède actuellement à une opération de désinfection sur place en enfonçant une résistance électrique dans la masse de matière.

Cette résistance chauffe localement la matière pour la désinfecter.

Malheureusement, l'incidence thermique pratiquée de cette manière s'avère totalement insuffisante, et cette opération ne change en rien l'état bactériologique du diffuseur d'air et de l'intérieur de la cuve du lit fluidisé.

Par ailleurs, les calories emmagasinées dans la masse importante de matière mettent environ 48 heures à se dissiper.

Or, la stérilisation complète d'une masse microgranuleuse s'avère peu commode et difficile à mettre en oeuvre.

La présente invention a pour but de stériliser et de désinfecter toute la matière en suspension active constituée des microsphères utilisées dans un lit fluidisé.

A cet effet, elle se rapporte à un groupe mobile de stérilisation pour la matière microgranuleuse d'un lit fluidisé sous la forme d'un caisson mobile à compartiment haut et bas, le compartiment bas renfermant un groupe frigorifique et une pompe de transfert en liaison par conduit souple à couplage rapide avec un ensemble mobile d'aspiration sur le lit, le compartiment haut délimitant une cuve de stérilisation à fond perméable à l'air dans laquelle est apportée la matière microgranuleuse par la pompe de transfert et à l'arrière un volume aéraulique présentant des moyens de conditionnement et de soufflage de l'air à travers la masse de matière à stériliser.

De nombreux avantages découlent de l'unité mobile de stérilisation selon l'invention, tels que ceux précisés ci-après :
. intervention de stérilisation rapide à proximité du lit fluidisé ;
. stérilisation complète à haut degré de désinfection ;
. fonctionnement en circuit fermé sans contact avec l'extérieur ;
. mobilité de l'unité ;
. le renouvellement s'effectue à travers l'appareil pour la sécurité de l'opérateur ; la masse étant enlevée du lit, on peut nettoyer et désinfecter celui-ci.

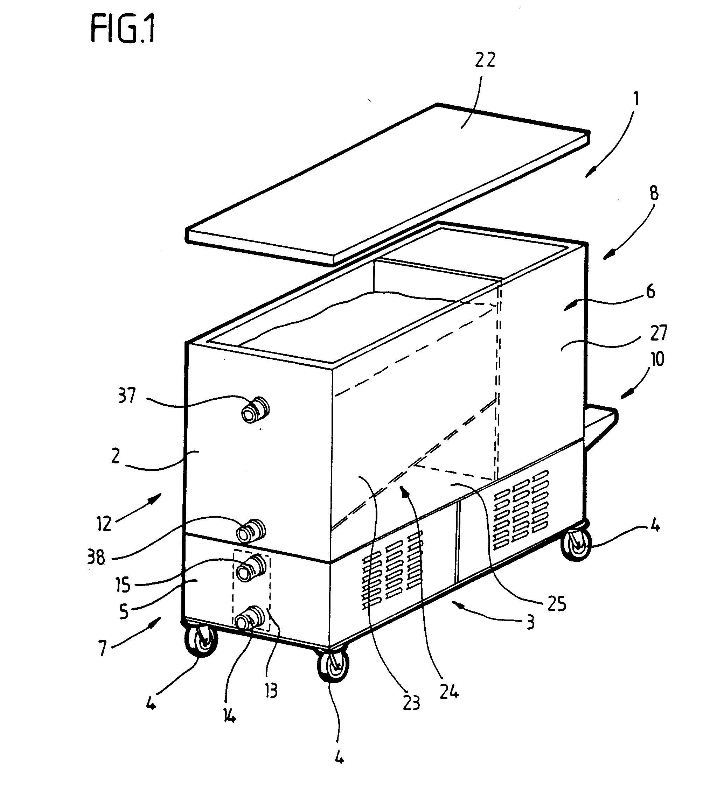

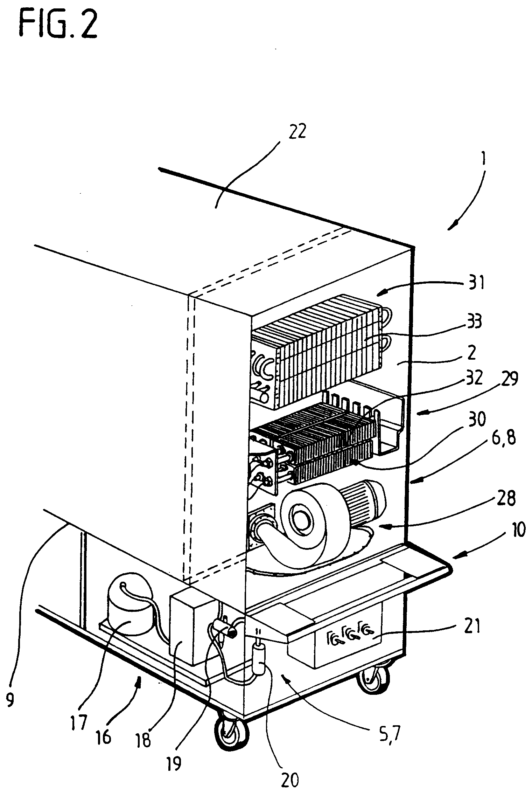

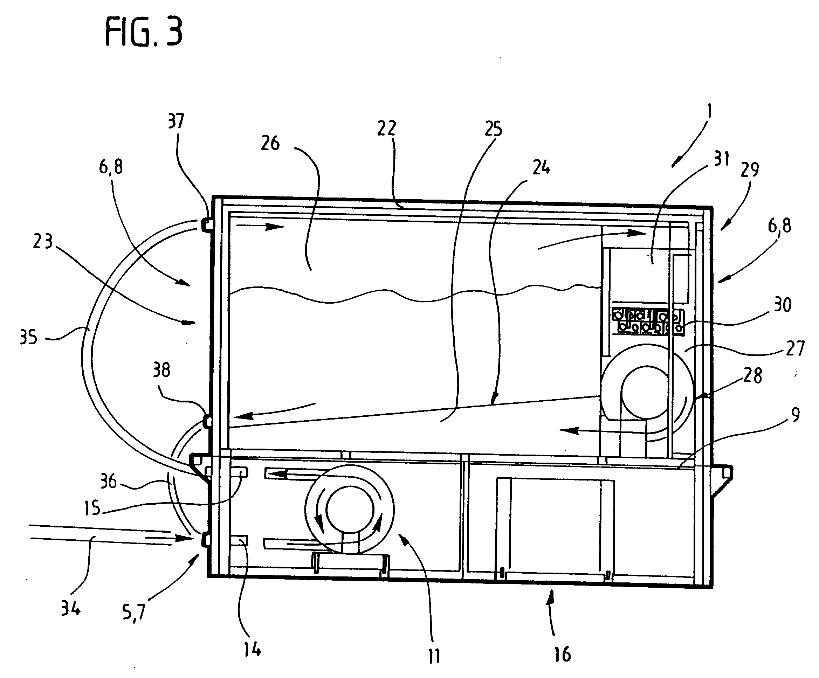

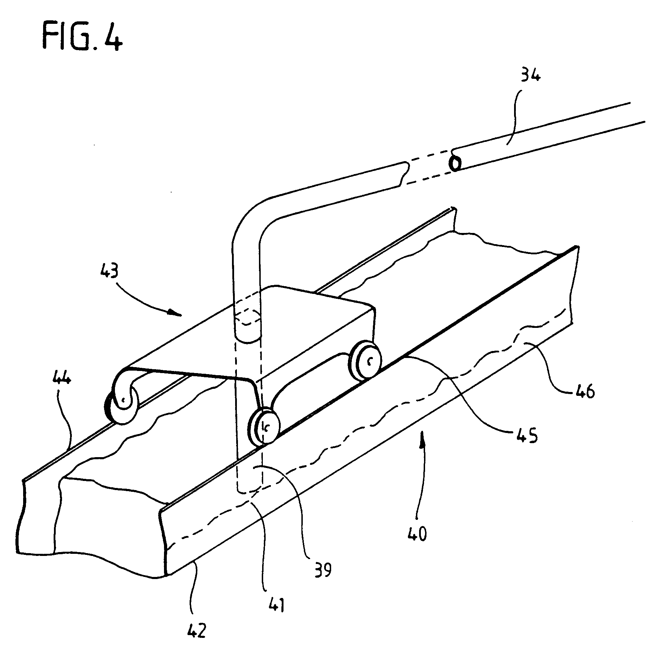

Les caractéristiques techniques et d'autres avantages de l'invention sont consignés dans la description qui suit, effectuée à titre d'exemple non limitatif sur un mode d'exécution en référence aux dessins accompagnants dans lesquels :
. la figure 1 est une vue en perspective simplifiée du groupe mobile de stérilisation selon l'invention, vue de trois quarts avant ;
. la figure 2 est une vue en perspective simplifiée du groupe mobile de stérilisation selon l'invention, vue de trois quarts arrière avec face arrière enlevée et partie inférieure latérale ouverte ;
. la figure 3 est une vue schématique en coupe longitudinale au niveau de la turbine de soufflage de l'unité ;
. la figure 4 est une vue en perspective de l'ensemble mobile d'aspiration.

L'appareil selon l'invention est un groupe mobile de stérilisation, comprenant un corps 1 sous la forme d'une coque ou d'un caisson 2 monté sur un chariot 3 équipé de quatre roulettes telles que 4.

Ce caisson est formé de deux compartiments superposés 5 et 6, respectivement bas et haut, constituant une unité de transfert 7 et une unité de stérilisation 8 séparées par une paroi 9.

Le caisson présente sur sa face arrière une poignée de manoeuvre 10.

Le groupe de transfert 7 comprend un organe moteur sous la forme d'une pompe de transfert 11, par exemple péristaltique, disposée longitudinalement, face avant en regard de la paroi frontale 12, précédée d'un bloc de raccordement rapide 13 comportant deux raccords à accouplement rapide 14 et 15 disposés par exemple sur la partie frontale, raccords pouvant être utilisés indifféremment comme entrée ou sortie.

Bien entendu, toute pompe existante ou à venir remplissant cette fonction pour ce type de produit peut convenir. On cite à cet effet, les pompes à vis, à engrenages, à membrane, à turbine...

Le compartiment bas 5 comprend également les éléments et organes suivants.

Un groupe frigorifique 16 se composant d'un compresseur 17, d'une batterie de condensation 18, d'un thermostat 19 et d'un filtre asséchant 20 est placé dans la partie arrière du compartiment bas, ainsi qu'un coffret de commande 21 destiné à coordonner les actions de commande du fonctionnement d'ensemble.

Le groupe frigorifique 16 est destiné au refroidissement de l'air dans un compartiment spécial, comme on le verra ci-après.

Le compartiment haut 6 est constitué d'un ensemble monocoque rigide entièrement calorifugé à couvercle 22 rapporté et présente une paroi arrière démontable.

Ce compartiment haut 6 formant l'unité de stérilisation 8 délimite une cuve de stérilisation 23 dans laquelle le produit microgranuleux est apporté et déversé puis prélevé par la pompe de transfert lors des phases de transfert entre le lit et le groupe de stérilisation.

La cuve de stérilisation 23 présente un fond 24 incliné ou horizontal. Il s'agit d'un contenant à fond perméable à l'air permettant de supporter une charge importante. Le fond 24 de la cuve de stérilisation délimite, avec la paroi de séparation 9, un volume inférieur d'insufflation 25.

Un volume intermédiaire de dégagement 26 est prévu en partie supérieure de la cuve pour l'échappement de l'air à l'extérieur ou sa réutilisation en recyclage.

Le compartiment haut 6 délimite également à l'arrière un espace aéraulique 27 renfermant des moyens de soufflage d'un flux d'air chaud de stérilisation, puis frais de refroidissement, dans le volume inférieur d'insufflation 25 pour traverser la masse microgranuleuse présente dans la cuve de stérilisation 23.

L'espace aéraulique 27 est en communication avec le volume de dégagement 26.

Les moyens aérauliques comprennent une turbine de soufflage 28 aspirant l'air provenant de l'extérieur et en recyclage celui s'échappant de la cuve de stérilisation 23 après passage sur un groupe de conditionnement d'air 29 occupant la partie supérieure de l'espace aéraulique.

Ce groupe de conditionnement d'air se compose de deux étages constitués d'une batterie chaude 30 et d'une batterie froide 31.

Pour la première, il s'agit de plusieurs résistances électriques blindées à ailettes telles que 32 et pour la seconde, de l'évaporateur 33 du groupe frigorifique 16 situé dans le compartiment bas.

Les transferts s'effectuent à l'aide de conduits souples d'amenée et de restitution 34 et de pontage 35 et 36 stérilisables, terminés par des coupleurs rapides venant s'adapter sur les raccords à accouplement rapide complémentaires 14,15 et 37,38 prévus sur la face avant du groupe de stérilisation au niveau de l'entrée et de la sortie de la pompe de transfert.

Le chargement et le déchargement de la cuve de stérilisation s'effectuent par pontage des entrées et sorties respectives de la cuve de stérilisation 23 et de la pompe de transfert 11, par les liaisons souples de pontage 35 et 36, détachables pour les besoins de stérilisation.

Selon la version de base, la désorption, c'est-à-dire le retour de la matière dans le lit, est assurée par la pompe de transfert 11.

En variante, un aspirateur de désorption peut être prévu en sortie pour la restitution de la matière traitée.

En vue de prélever la matière dans le lit fluidisé, on prévoit un tube d'aspiration 39 plongeant dans la matière contenue dans la cuve 40 jusqu'à une distance prédéterminée 41 du fond 42. Ce tube est porté par un chariot 43 qui se déplace au-dessus et le long du lit, par exemple en roulant sur les bords longitudinaux du lit 44 et 45.

La distance prédéterminée d'aspiration par rapport au fond 42 est maintenue pour éviter l'aspiration des agglomérats 46 de matière souillée tombés sur le fond.

On décrira maintenant le mode opératoire, c'est-à-dire le procédé de chargement/déchargement de la cuve de stérilisation et celui de stérilisation.

Le groupe mobile de stérilisation est amené à proximité du lit fluidisé par roulement.

Il est raccordé par le conduit de transfert 34 au lit fluidisé au moyen du chariot mobile d'aspiration 43 se terminant à l'intérieur du lit par le tube d'aspiration 39.

Le chariot se déplace le long de chacun des bords longitudinaux 44 et 45 du lit afin d'aspirer toute la matière microgranuleuse libre en laissant les agglomérats 46 au fond.

L'aspiration s'effectue jusqu'à évacuation totale des microsphères libres, c'est à dire celles situées au-dessus de la garde existant par rapport au fond correspondant au volume de matières agglomérées à évacuer et à remplacer.

Le transfert de la matière s'effectue automatiquement par le travail de la pompe.

Pour la fin d'aspiration, l'opérateur travaillera manuellement jusqu'à admission totale de toute la matière libre, puis débranchera l'arrivée dans la cuve de stérilisation.

Commence alors la phase de stérilisation qui s'effectue de préférence en circuit fermé par recyclage de l'air chauffé ou réchauffé par la batterie chaude et insufflation à travers le fond de la cuve de stérilisation à une tepérature suffisante, par exemple de l'ordre de 90-100 °C.

Le temps de soufflage par air chaud délivré par la batterie chaude est limité à une durée suffisante pour la stérilisation complète et efficace de tout le volume, par exemple quarante minutes environ pour un volume moyen, après une phase transitoire de montée en température.

Ensuite débute la phase de refroidissement réalisé également par insufflation d'air à travers la matière par passage sur la batterie froide, qui a pour but de ramener la masse des microsphères à une température d'environ par exemple 35 °C.

Les microsphères sont alors retransférées dans la cuve du lit fluidisé par inversion de l'aspiration de la pompe de transfert ou inversion des branchements entrée/sortie et cuve/pompe en vue du rechargement du lit en matière.

Comme déjà indiqué, le lit fluidisé aura pu entre temps être nettoyé et désinfecté.

A chaque stérilisation, il sera procédé à un appoint de microsphères neuves correspondant au volume des agglomérats non aspirés et évacués comme déchets.

Il est bien entendu qu'au-delà des moyens décrits, diverses modifications évidentes et variantes simples entrent dans le cadre de la présente invention.

## Revendications

1. Groupe mobile de stérilisation pour une matière microgranuleuse, notamment celle utilisée dans un lit fluidisé, se présentant sous la forme d'un caisson qui se compose d'un compartiment bas et d'un compartiment haut délimitant une cuve de stérilisation à fond perméable à l'air, caractérisé en ce que le compartiment bas renferme un groupe frigorifique et une pompe de transfert en liaison par conduit à couplage rapide avec un ensemble mobile d'aspiration sur le lit, ladite pompe de transfert apportant ladite matière microgranuleuse dans ladite cuve, à l'arrière de laquelle se situe un volume aéraulique présentant des moyens de conditionnement et de soufflage de l'air à travers la masse de matière présente dans la cuve en vue de sa stérilisation.

2. Groupe mobile de stérilisation selon la revendication 1, caractérisé en ce que la masse de matière est chargée et déchargée dans et hors de la cuve par la pompe de transfert.

3. Groupe mobile de stérilisation selon la revendication 1, caractérisé en ce que les liaisons entre la pompe de transfert et la cuve de stérilisation sont extérieures et amovibles.

4. Groupe mobile de stérilisation selon les revendications précédentes, caractérisé en ce que l'ensemble d'aspiration présente un chariot qui se déplace le long des bords longitudinaux du lit et porte le tube d'aspiration.

5. Groupe mobile de stérilisation selon la revendication 4, caractérisé en ce que l'extrémité du tube d'aspiration est située à une distance prédéterminée du fond de la cuve du lit.

6. Groupe mobile de stérilisation selon la revendication 5, caractérisé en ce que la distance prédéterminée du fond est égale à l'épaisseur d'un volume correspondant à la couche d'agglomérats présents en fond de cuve.

7. Groupe mobile de stérilisation selon l'une quelconque des revendications précédentes, caractérisé en ce que le fond de la cuve de stérilisation est plat.

8. Groupe mobile de stérilisation selon l'une quelconque des revendications précédentes, caractérisé en ce que le fond de la cuve de stérilisation est incliné descendant de haut en bas, de l'arrière vers l'avant.

9. Procédé de stérilisation d'une matière microgranuleuse présente dans un contenant, notamment la cuve d'un lit fluidisé, caractérisé en ce que l'on transfère par aspiration à travers une liaison fluidique le produit jusqu'à la cuve de stérilisation du groupe mobile de stérilisation revendiqué ci-dessus par une pompe de transfert, que l'on porte par un groupe aéraulique à une température de stérilisation la masse du produit pendant une durée supérieure à la durée théorique de stérilisation et que l'on refroidit la masse du produit avant de la transférer à nouveau dans la cuve du lit.

10. Procédé selon la revendication 9, caractérisé en ce que le chauffage puis le refroidissement de la matière s'effectuent par insufflation dans la masse de celle-ci, à travers le fond de la cuve de stérilisation, d'un flux d'air porté à une température de stérilisation, puis d'un flux d'air de refroidissement à une température plus basse.

11. Procédé selon la revendication 10, caractérisé en ce que l'on recycle l'air de stérilisation à travers le groupe aéraulique.
